# EUROPEAN PATENT SPECIFICATION

(11) **EP 2 352 465 B1**
(45) Date of publication and mention of the grant of the patent: **21.03.2018**
(21) Application number: 09789195.6
(22) Date of filing: 24.08.2009
(51) Int. Cl.: A61F 2/88

(54) **MULTI-SECTION STENT**
STENT MIT MEHREREN ZONEN
PROTHÈSE ENDOVASCULAIRE EN PLUSIEURS PARTIES

(30) Priority: 27.08.2008 US 92287 P
(43) Date of publication of application: 10.08.2011
(73) Proprietor: Cook Medical Technologies LLC, Bloomington, IN 47404 (US)
(72) Inventor: FISCHER, Frank J., Bloomington IN 47401 (US); LOWINGER, Johan M., Bloomington IN 47401 (US)
(74) Representative: Garratt, Peter Douglas
(86) International application number: PCT/US2009/004806
(87) International publication number: WO 2010/024868

(56) References cited:
- WO-A-03/086237
- WO-A-2004/058100
- WO-A-2006/108010
- US-A1- 2003 212 450

## Description

### BACKGROUND

Peripheral vascular disease is characterized by insufficient blood flow in the extremities. This insufficient blood flow is often the result of stenosis, the occlusion of the blood vessels. Stenosis associated with peripheral vascular disease may be caused by atherosclerosis, the occlusion of blood vessels through the buildup of fatty deposits along the arterial walls, or a combination of atherosclerosis and thrombosis, the occlusion of blood vessels by clotted blood.

Over time, arterial disease may result in acute ischemia, the complete cut-off of a blood vessel, or chronic ischemia, the re-routing of blood flow through collateral vessels. Some persons suffering from arterial disease experience claudication - the atherosclerosis of the blood vessels in the lower extremities. Persons suffering from claudication may lose the ability to walk due to the crippling of the feet and legs. Ischemia and chronic ischemia are also known to cause pain and other forms of physical impairment.

Stents are commonly used to treat peripheral vascular disease. Stents may be inserted into a diseased blood vessel and used to keep the lumen of the blood vessel open. This is accomplished by inserting a compressed stent into the portion of the blood vessel affected by atherosclerosis and/or thrombosis. Once inserted into the desired location of the vessel, the stent is expanded. The expansion of the stent may be accomplished by either using a self-expanding stent or a balloon-expandable stent.

A self-expanding stent must be compressed in order to be inserted into a blood vessel. The self-expanding stent will naturally return to its original expanded position once the compressing pressure is removed. In contrast, a balloon expandable stent begins in a compressed state. Once the stent is in place, a balloon in the lumen of the stent is filled with saline to expand the stent. The balloon-expandable stent will retain the expanded size even after the balloon is removed.

Stents may also be used in combination with other components to treat a number of medical conditions. For example, stent-graft assemblies are commonly used in the treatment of aneurysms. An aneurysm is an abnormal widening or ballooning of a portion of an artery. Generally, this condition is caused by a weakness in the blood vessel wall. High blood pressure and atherosclerotic disease may also contribute to the formation of aneurysms. Common types of aneurysms include aortic aneurysms, cerebral aneurysms, popliteal artery aneurysms, mesenteric artery aneurysms, and splenic artery aneurysms. However, it is also possible for aneurysms to form in blood vessels throughout the vasculature. If not treated, an aneurysm may eventually rupture, resulting in internal hemorrhaging. In many cases, the internal bleeding may be so massive that a patient can die within minutes of an aneurysm rupture. For example, in the case of aortic aneurysms, the survival rate after a rupture can be as low as 20%.

An aneurysm may be treated with a stent-graft by implanting a stent-graft in the blood vessel across the aneurysm using conventional catheter-based placement techniques. The stent-graft treats the aneurysm by sealing the wall of the blood vessel with a generally impermeable graft material. Thus, the aneurysm is sealed off and blood flow is kept within the primary passageway of the blood vessel. Increasingly, treatments using stent-grafts are becoming preferred since the procedure results in less trauma and faster recuperation than conventional surgical techniques for treating aneurysms.

Many stents known in the art are formed of a lattice of metallic material. Lattice stents may be divided into two groups based on the cells formed by the lattice: open-cell stents and closed-cell stents. Open-cell stents typically feature rings of metal connected together by substantially longitudinally-running connecting members. The substantially longitudinally-running connecting members are often called tie bars or bridges. The rings may take a variety of shapes, e.g., a zigzag configuration. The tie bars may also vary in their length, number, and whether they run parallel with the length of the stent or at an angle. In open-cell stents, the frequency of tie bars connecting the rings is such that long cells are formed between the rings of the stent. Thus, an open-cell stent has a high ratio of rings to tie bars relative to a closed-cell stent. As a result, the cells of an open cell stent generally have a longitudinal length that is substantially smaller than their circumferential length. Open-cell stents may be advantageous because of their high flexibility and conformability to the vessel walls.

In a closed-cell stent, the metal lattice of the stent forms cells in which the longitudinal length of the cells is approximately equal to the circumferential length of the cells. The frequency of bars connecting the rings in a closed-cell stent is generally higher than an open-cell stent. Closed-cell stents typically have a metallic structure that forms a plurality of identical openings. For example, one type of closed-cell stent is a metallic stent with a plurality of identical-diamond shaped openings. Closed-cell stents provide strong radial strength and good plaque coverage. The ratio of tie bars to rings in a closed-cell stent is high relative to an open-cell stent.

To facilitate stent implantation, stents are normally loaded on the end of a catheter in a low profile, compressed state. A sheath is placed over the stent and catheter to maintain the stent in the low profile, compressed state. The catheter is then used to guide the stent to the portion of the vessel to be treated. Once the stent is positioned adjacent the portion of the blood vessel to be treated, the stent is released by pulling, or withdrawing, the sheath rearward. Normally, a stop member or other feature is provided on the catheter to prevent the stent from moving rearward with the sheath. After the stent is released from the retaining sheath, a self-expanding stent will spring radially outward to an expanded diameter until the stent contacts and presses against the vessel wall. A coiled stent must be twisted for it to expand. Once the stent is expanded, the catheter used to insert it is withdrawn, leaving the stent in place within the vessel.

The forces bearing on the knee present difficulties for the use of stents to treat peripheral artery disease in the blood vessels of the knee. The superficial femoral artery (SFA) is one artery in the knee that is commonly affected by peripheral artery disease. Depending on whether a person is standing, sitting, or walking, the SFA is subject to axial (stretching), radial (bending), and torsional (twisting) forces. The SFA and other blood vessels in the knee are subject to all of these forces at high magnitudes and at high frequency.

The stents that are currently used in the SFA and other blood vessels in the knee, such as the popliteal, tend to kink and fracture at a higher rate than stents in other blood vessels throughout the body. The reason for the high kinking and fracture rate in the knee is due to the variety of forces that bear on the vessel and the frequency and magnitude of their application. Kinking occurs when a stent is bent radially beyond its tolerance. A kinked stent will obstruct blood flow. Stent fractures may be caused by the application of excessive axial, radial, or torsional force. A fractured stent may irritate the walls of the blood vessel, leading to abnormal cell growth, known as hyperplasia. Moreover, stent fractures are associated with restenosis of blood vessels. Thus, the inventors believe that there is a need for a stent that is better equipped to withstand various forces to successfully treat peripheral vascular disease in the knee.

With regard to the prior art, reference is firstly directed to WO03/086237, which discloses a stent that comprises a plurality of segments, including at least one segment which is in the form of a coil and at least one segment which is in a form other than a coil and which is balloon expandable or self-expandable.

The document teaches that the disclosed stents might find use in the cerebral arteries as well as in the coronary arteries, the peripheral arteries and the arteries of the neck. However, it also teaches that the stents are not limited to use in the vascular system and may also be employed in other body structures, including but not limited to arteries, veins, biliary ducts, urethras, fallopian tubes, bronchial tubes, the trachea, the esophagus, the prostate and the bowel. In a specific example, the document teaches that the stents may be used interarterially in the brain, deployed across the neck of an aneurysm as well as in occlusions in bodily vessels

Reference is additionally directed to WO2004/058100, which discloses an implantable vascular prosthesis. The document teaches that the disclosed stent may be configured for use in a wide range of applications, such as treating aneurysms, maintaining patency in a vessel, and allowing for the controlled delivery of therapeutic agents to a vessel wall. The prosthesis comprises one or more helical sections coupled to one or more anchoring sections having a generally zig-zag or cell-like configuration. The prosthesis is configured to conform to a vessel wall without substantially remodeling the vessel, and further is configured to be precisely deployed in a vessel without shifting or foreshortening during deployment.

Reference is further directed to WO2006/108010, which discusses issues that may be experienced when stenting the superficial femoral artery. The document proposes a flexible stent structure including a plurality of axially spaced strut portions. These strut portions define generally tubular axial segments of the stent that are radially expandable. A helical portion is interposed axially between two strut portions and has a plurality of helical elements connected between circumferentially spaced locations on the two strut portions. The helical elements extend helically between those locations and the length of a helical element is sufficient so that, when the stent is in a radially expanded state, it can simultaneously withstand repeated axial compression or expansion and bending.

### BRIEF SUMMARY

Aspects of the invention are set out in the appended claims.

There is described a multi-section stent comprising: a first tubular section having a first end and a second end; a second tubular section connected to said first end of said first tubular section; and a third tubular section connected to said second end of said first tubular section, wherein each tubular section comprises at least one segment defining at least one cell that extends longitudinally and circumferentially of the device, and wherein the ratio of the circumferential length to the longitudinal length of said segment is greater in said first tubular section than in either of said second tubular section or said third tubular section.

An example of a multi-section tubular device comprises a device wherein the first tubular section, the second tubular section, and the third tubular section are nitinol.

Another example of a multi-section tubular device further comprises a radio-opaque substance contained on at least one of the at least substantially longitudinally running connector segments linking two adjacent sections of the multi-section tubular device together.

Another example of a multi-section tubular device comprises a radio-opaque substance coated on at least one of the at least substantially longitudinally running connector segments linking two adjacent sections of the multi-section tubular device together.

The multi-section tubular device comprises: a first tubular section having a first end and a second end; a second tubular section connected to the first end of the first tubular section; and a third tubular section connected to the second end of the first tubular section, wherein each of the first tubular section, the second tubular section, and the third tubular section is formed of a lattice, the lattices each having a plurality of ring segments
extending circumferentially and at least three connector segments linking each of the ring segments together with each adjacent ring segment, wherein the first tubular section has a substantially lower ratio of the connector segments to ring segments than the second tubular section, and the first tubular section has a substantially lower ratio of the connector segments to ring segments than the third tubular section.

### BRIEF DESCRIPTION OF THE DRAWINGS

Figure 1 is a partial perspective view of an example of a multi-section tubular device.
Figure 2 is a partial perspective view of the tubular device of Figure 1.
Figure 3 is a perspective view of another example of a multi-section tubular device.
Figure 4 is a partial perspective view of the tubular device of Figure 3.
Figure 5 is a partial perspective view of a multi-section tubular device according to an embodiment of the present invention.
Figure 6 is a partial perspective view of a further example of a tubular device.
Figure 7 is a partial perspective view of a still further example of a tubular device.

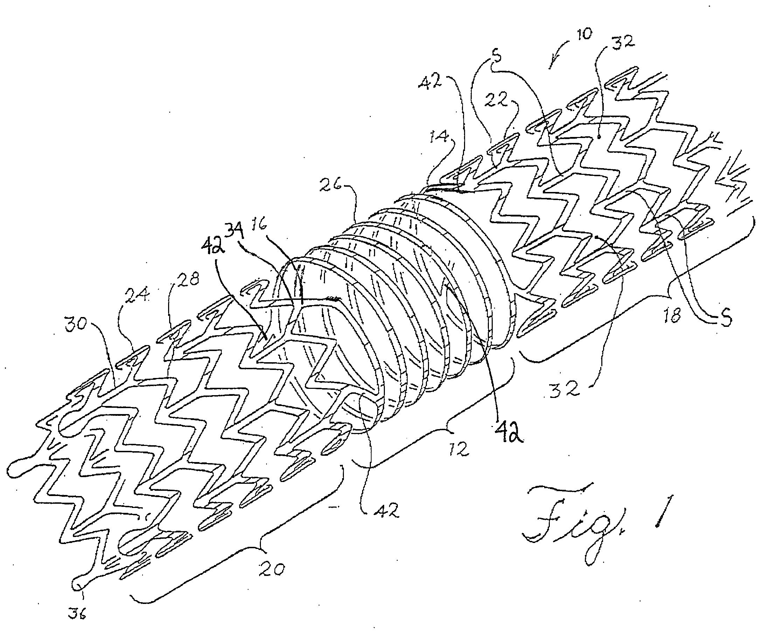

Figures 1 to 4, 6 and 7 do not show embodiments of the invention.

### DETAILED DESCRIPTION OF THE DRAWINGS AND THE PRESENTLY PREFERRED EMBODIMENTS

Referring now to the drawings, and particularly to Figure 1, an example of a multi-section tubular device 10 is shown. The tubular device 10 includes a first tubular section 12 having a first end 14 and a second end 16. A second tubular section 18 is connected to the first end 14 of the first tubular section 12. A third tubular section 20 is connected to the second end 16 of the first tubular section 12. As shown, the first tubular section 12 is formed of a coil 26. The second tubular section 18 is formed of a first lattice 22. The third tubular section 20 is formed of a second lattice 24.

The multi-section tubular device may be inserted into an occluded or damaged blood vessel. Once expanded, the tubular device creates an opening in the otherwise occluded vessel to allow for the passage of blood. The coiled first tubular section 12 is very flexible and not prone to kinking or fracturing even when subject to a wide variety of different types of force, including axial (stretching), radial (bending), and torsional (twisting) forces. The lattice of the second tubular section 18 and the third tubular section 20 provides high radial force, but is less axially flexible and thus less kink and fracture-resistant. Thus, it is preferable that the tubular device 10 be placed in the vessel such that the first tubular section 12 is subject to the most intense forces.

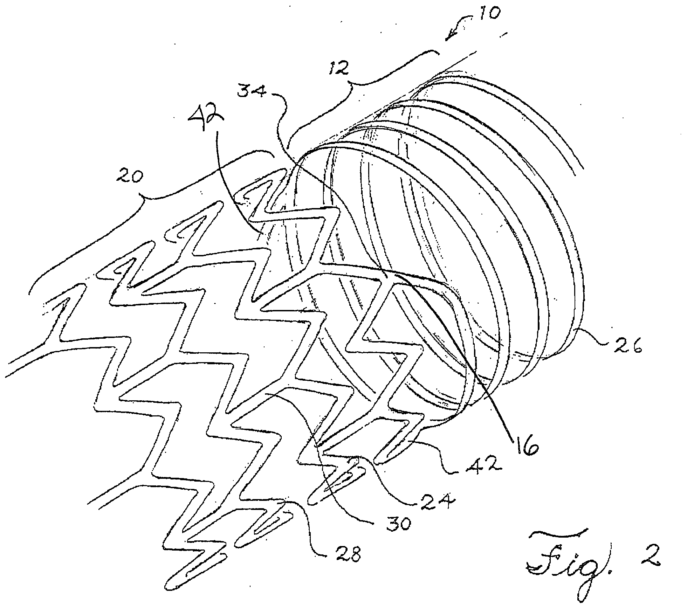

In the example of a tubular device in Figure 1 , the lattice of the second tubular section 18 and the third tubular section 20 is formed of a series of ring segments 28 extending circumferentially around the tubular device 10. The ring segments 28 shown in Figures 1 and 2 have a zigzag pattern such that the ring segments 28 form a series of "Z"s. Any other pattern known in the art may be used instead of or in conjunction with the zigzag pattern shown in Figure 1.

Figure 1 illustrates an example of a tubular device in which each ring segment 28 is connected to the two adjacent ring segments 28 by a plurality of longitudinally-running connector segments 30. The longitudinally-running connector segments 30 are spaced such that they do not form a continuous axial line along the length of any section of the tubular device 12. Instead, the longitudinally-running connector segments 30 are placed such that they are offset from one another along the longitudinal axis of the tubular device 10. The connector segments 30 may also be spaced in any other way known in the art.

The circumferentially-running ring segments 28 and longitudinally-running connector segments 30 form between them a plurality of cells 32. In the lattice of the second tubular section 18 and the lattice of the third tubular section 20, the cells formed by the ring segments are relatively long in the circumferential direction. As shown in Figure 1, the circumferential length of the cells may be approximately three times as great as the longitudinal length of the cells. Lattices with cells having a substantially greater circumferential length than a longitudinal length are generally known as open-cell stents.

In the example of a tubular device shown in Figure 1, the first end 14 of the first tubular section 12 is connected to the nearest ring segment of the second tubular section 18. Likewise, the second end 16 of the first tubular section 12 is connected to the nearest ring segment of the third tubular section 20. The connection between the second tubular section 16 and the first tubular section 12 is shown in Figure 2. As shown, the second end 16 may be connected to a point 34 formed by the zig-zag-shaped ring segment 28 of the third tubular section 20. The first end 16 is preferably blended with this point. As shown in Figure 1, in some constructions it may be preferable to have at least two connector segments 42 link the first tubular section 12 with the third tubular section 20, thereby forming a total of three connections between the two sections. The connection between the first tubular section 12 and the second tubular section 18 may be formed of a similar structure. Although at least three connections between each adjacent section of the tubular body may be preferable in some constructions, other constructions may have one connection or two connections.

The connector segments 42 linking the coiled first tubular section 12 to the second tubular section 18 are preferably angled in the same direction as the coiled first tubular section 12. This may be advantageous because it allows the connector segments 42 to flex in the same direction as the coil 26, thereby reducing the stress on the connector segments 42 when the tubular device 10 is compressed. It also may allow the connector segments 42 to flex when the stent is exposed to the high axial, radial, and torsional forces of the knee. The connector segments 42 may also run in a substantially longitudinal direction or be angled in a direction opposite to the angle of the coil of the first tubular section 12.

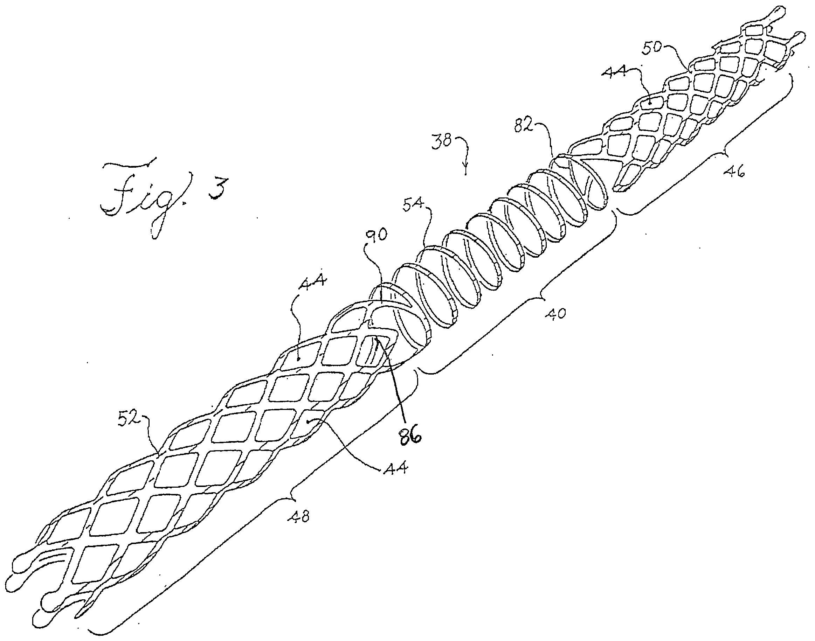

Figure 3 shows a further example of a tubular device 38 that has a second 46 and third tubular section 48 formed of a lattice of cells. Each cell 44 has a longitudinal height that is approximately equal to its circumferential height. In this example of a tubular device, the second tubular section 46 may be characterized as being formed of a first lattice 50 with a closed-cell structure. Likewise, the third tubular section 48 may be characterized as being formed of a second lattice 52 with a closed-cell structure. The lattices shown in Figure 2 are formed of cross-hatching such that the lattices form a plurality of identical diamond-shaped cells. Any other form of closed-cell lattice known in the art may be used in lieu of or in conjunction with the cross-hatched lattices shown in Figure 2.

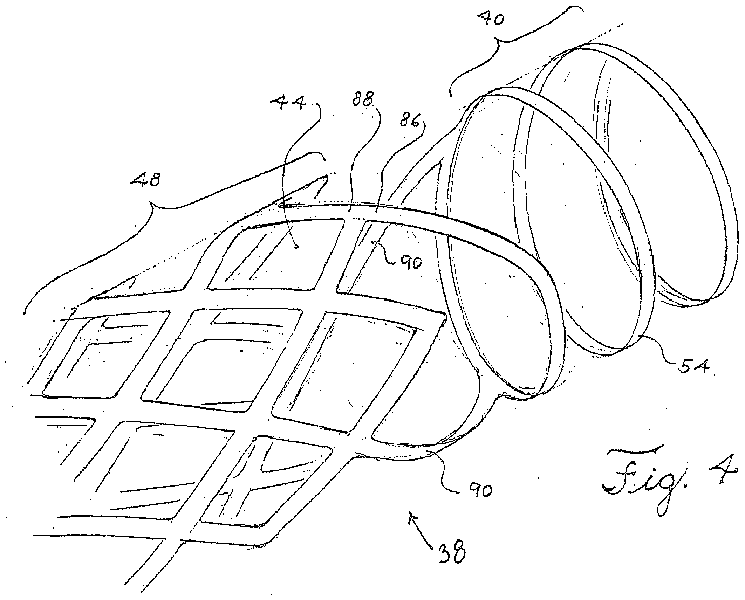

Figure 4 shows the connection between the coiled first tubular section 40 and the adjacent third tubular section 48 of the tubular device 38 of Figure 3. As shown in Figure 3, the coil 54 of the first tubular section 40 has a first end 82 and a second end 86. As shown in Figure 4, the first tubular section 40 is formed of a coil 54. The second end 86 of the coil 54 of the first tubular section 40 may be connected to a point 88 of one of the diamond-shaped cells 44 in the third tubular section 48. In addition, the coiled first tubular section 40 is connected to the third tubular section 48 by two connector segments 90, thereby forming three points of attachment between the first 40 and third tubular sections 48. The first end 82 of the first tubular section 40 may be attached to the second tubular section 46 in the same manner.

As shown in Figure 4, the connector segments 90 linking the first 40 and third tubular sections 48 of the example of a tubular device shown in Figure 3 are preferably angled in the same direction as the coil 54 of the first tubular section 40. This may be advantageous because it allows the connector segments 90 to flex in the same direction as the coil 54, thereby reducing the stress on the connector segments 90 when the tubular device 38 is compressed. It also may allow the connector segments 90 to flex when the stent is exposed to the high axial, radial, and torsional forces of the knee. The connector segments 90 may also run in a substantially longitudinal direction or be angled in a direction opposite to the angle of the coil 54 of the first tubular section 40.

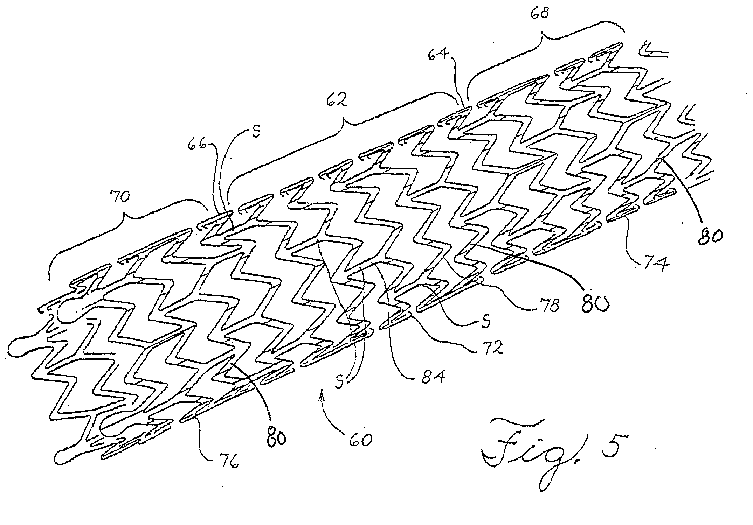

Figure 5 illustrates a multi-section tubular device 60 according to an embodiment of the present invention. According to the embodiment, the first tubular section 62 is formed of an open-cell first lattice 72. The first tubular section 62 of the tubular device 60 shown in Figure 5 has a first end 64 and a second end 66. A second tubular section 68 is connected to the first end 64 of the first tubular section 62. A third tubular section 70 is connected to the second end 66 of the first tubular section 62. The second tubular section 68 forms a second lattice 74 and the third tubular section 70 forms a third lattice 76. The first, second, and third lattices are each formed of a plurality of ring segments 80. Adjacent ring segments 78 within each of the three lattices are connected together by at least three connector segments 84.

In Figure 5, the number of connector segments 84 connecting adjacent ring segments 78 in the second tubular section 68 and the third tubular section 70 is higher per ring segment 78, than the number of connector segments 84 connecting adjacent ring segments 78 in the first tubular section 62. This results in a lower ratio of connector segments 84 to ring segments 78 in the first tubular section 62 than in either the second tubular section 68 or the third tubular section 70. For example, the ring segments 78 of the second tubular section may have an average of five connector segments 84 linking any two adjacent ring segments 78. There may be only 3 connector segments 84 linking any two adjacent ring segments 78 in the first tubular section 62. In the foregoing example, the ratio of connector segments 84 is lower in the first tubular section 62 than in the second tubular section 68. Figure 5 illustrates an embodiment having fewer connector segments in the first tubular section than the second and third tubular sections.

In some variations of the embodiment shown in Figure 5, there are no more than three connector segments 84 connecting any single ring segment 78 to an adjacent ring segment 78 in the first tubular section 62. It may be preferable for some embodiments that the number of connector segments 84 in the first tubular section 62 be greater per ring segment 78 near the first end 64 and the second end 66 of the first tubular section 62 and that the number of connector segments 84 per ring segment 78 decrease toward the center of the first tubular section 62. This may be advantageous because it provides the greatest axial and radial flexibility at the center of the first tubular section 62. The device will be progressively less axially and radially flexible, but have greater radial force, toward the first end 64 and the second end 66 of the first tubular section 62. This may enable the device to exert the maximum radial force to keep the vessel open while also ensuring that it is sufficiently flexible to withstand the forces that bear on blood vessels in the knee.

In some embodiments the offset between the connector segments 84 in the open-cell lattice may create a spiral pattern as shown in the first tubular section 62 in Figure 5. The connector segments 84 labeled "S" in Figure 5 illustrate this spiral pattern. This type of spiraling pattern may also be used in an open-cell lattice of the second tubular section or third tubular section in constructions having a coil for the first tubular section. In constructions combining a coiled first tubular section and a spiraling open-cell lattice, such as the example of a tubular device depicted in Figure 1 , the spiral pattern of the connector segments 30 preferably follows the same spiral direction as the coil 26 of the first tubular section 12. The connector segments 30 forming one such spiral pattern are labeled "S" in the second tubular section 18 of Figure 1. This allows the connector segments to bend in the same direction as the coil twists and thus provides high flexibility. However, any other pattern of connector segments 30 known in the art may be used.

In Figures 1 and 5, the connector segments connecting adjacent ring segments within the second and third tubular sections run longitudinally - that is, they run parallel to the longitudinal axis of the tubular body. In other embodiments, the connector segments will run substantially longitudinally. In embodiments with substantially longitudinally-running connector segments, the segments may run at an angle that is not parallel to the longitudinal axis of the tubular body. A substantially longitudinally-running connector segment may also feature any variety of bend, curve or other configuration. Thus, although the substantially longitudinally-running connector segments connect the ring segments in the longitudinal direction, the substantially longitudinally-running connector segment may not run directly parallel to the longitudinal axis of the tubular body and portions of the substantially longitudinally-running connector segment may even run circumferentially. It is preferable that the connector segments run at least substantially longitudinally. That is, it is preferable that the segments run either longitudinally or substantially longitudinally.

The examples of tubular devices shown in Figures 1, 2, 3, 4, and 6 each feature a coiled first tubular section that is connected to each adjacent tubular section via three direct connections: one formed by the end of the coil and two formed by connector segments. However, in some constructions having a coiled first tubular section, there may be only one direct connection between the coiled first tubular section and each adjacent tubular section. In these constructions, the only direct connections between the coiled first tubular section and the adjacent tubular sections are formed by the ends of the coil.

In constructions featuring a coiled first tubular section with only one direct connection between the first tubular section and adjacent tubular section, additional connections may optionally be formed by sutures. For example, the sutures may loop through the cells of the second tubular section and over the nearest full ring of the coil of the first tubular section. The sutures may be formed of one single piece of suture material, which is looped back and forth through the cells and over the ring of the coil several times. In constructions having one single piece of suture material, the two loose ends of the suture material may be tied together to secure the suture. The two loose ends may also be independently knotted to the tubular body. In the alternative, the sutures may be formed of several shorter pieces of suture material, which are knotted off periodically. The sutures may be formed of any biocompatible material known in the art. It is preferable that constructions having a coil with only one direct connection between the sections also include at least two sutures connecting the adjacent sections. However, other constructions may have only one suture or no sutures.

Sutures are a particularly advantageous method of attaching adjacent tubular sections in constructions featuring a coiled first tubular section. The coiled first tubular section requires torsional (twisting) force to compact the stent while the lattice of second and third tubular sections requires a compressive force to compact the stent. Likewise, the different tubular sections of the stent require different types of force for expansion. The lattice of the second and third tubular sections requires radial force - often obtained by using a self-expanding stent. In contrast, the coiled first tubular section requires twisting. The loops of the sutures may slide along the coil as the coil is twisted and the lattice contracts and expands. The use of sutures which may easily slide, bend, and twist to accommodate both torsional and compressive forces may facilitate loading the stent in a compressed state on an insertion device and also expanding the stent after it is inserted into a body.

Sutures may be used in lieu of connector segments or in addition to connector segments to connect adjacent tubular sections in any embodiment or example. Although sutures are particularly preferable in illustrative constructions having only one direct connection between the coiled first tubular section and the adjacent tubular sections, they may also be used in addition to direct connections formed by connector segments. Sutures may also be used in lieu of, or in addition to, connector segments to connect adjacent tubular sections in embodiments according to the present invention having three tubular sections each formed of a lattice similar to the one shown in Figure 5.

As shown in Figure 1 , the ends of the second tubular section and the third tubular section that are not attached to the first tubular section may feature rounded tips 36 extending from the points of the zigzags of the ring segments 28. These rounded tips 36 prevent the ends of the tubular device 10 from damaging the vessel walls once the tubular device 10 is in place. They also may serve as markers to aid in placement of the tubular device 10. When used as a marker, the rounded tips are embedded with a small quantity of a radio-opaque substance such as gold. The rounded tips may also be coated rather than embedded with the radio-opaque substance. In some embodiments according to the present invention, it may be preferable to use both embedding and coating together. The radio-opaque substance serves as a marker that enables physicians to locate the end of the stent for purposes of insertion or during routine follow-up procedures. Figures 3, 5, 6, and 7 also illustrate the use of rounded tips. Other embodiments will not have rounded tips at the ends of the second and third tubular sections. Still other embodiments may feature rounded tips that do not function as markers.

In other embodiments it may be preferable to use a radio-opaque substance such as gold along a junction between two adjacent the sections of the multi-section tubular device. The radio-opaque substance may be embedded inside the stent or it may be coated on the stent. The use of a radio-opaque substance at the juncture between two adjacent sections may facilitate placing the tubular device in a patient's blood vessel such that the flexible first section is located in the portion of the vessel subject to the highest radial, axial and torsional forces. In embodiments having a radio-opaque substance marking the sections of the multi-section stent, it is preferable that the radio-opaque substance be embedded in or coated on one or more of the connector segments between adjacent sections of the multi-section stent. A connector segment having a radio-opaque marker embedded within it may be somewhat thicker than other connector segments. It may be advantageous to utilize a radio-opaque coating in some embodiments because the coating may not require that the stent be substantially thicker to accommodate the radio-opaque substance. A radio-opaque substance may also be embedded in or coated on in a ring segment or a portion of a coil.

Figures 1 , 3, and 5 show tubular devices in which the second tubular section and third tubular sections are formed of a lattice with a similar structure. For example, Figure 1 shows a second tubular section 18 and a third tubular section 20 that are each formed of an open-cell lattice with a zigzag pattern. However, in some illustrative constructions, the lattice of the second tubular section and the third tubular section may differ. For example, in some illustrative constructions, the second tubular section may have an open-cell lattice while the third tubular section may have a closed-cell lattice. In addition, a single tubular device may feature two different types of open-cell lattices for the second and third tubular sections. A single tubular device may also feature two different types of closed-cell lattices.

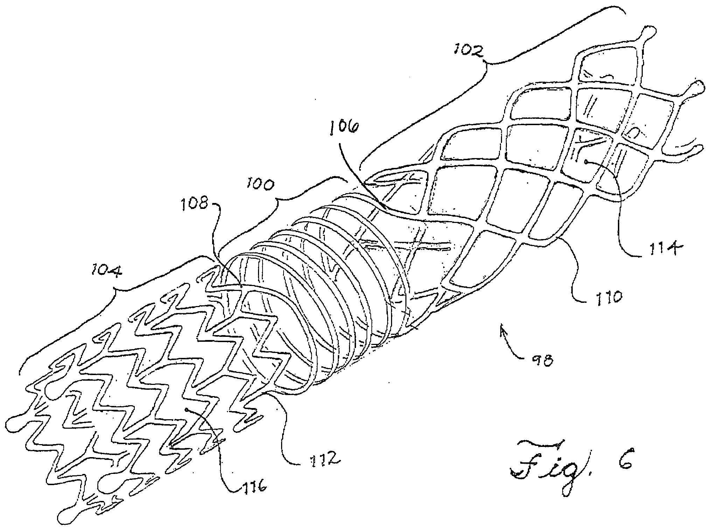

Figure 6 illustrates an example of a multi-section tubular device 98 having a coil and two different types of lattice. The multi-section tubular device 98 features a coiled first section 100, a second section 102, and a third section 104. The first end 106 of the coiled first section 100 is connected to the second section 102. The second end 108 of the coiled first section 100 is connected to the third section 104. The second section 102 forms a first lattice 110 and the third section 104 forms a second lattice 112. The first lattice 110 is comprised of cells 114 each having a longitudinal length that is approximately equal to the circumferential length of the cell. In contrast, the second lattice 112 is comprised of cells 116 each having a longitudinal length that is substantially smaller than the circumferential length of the cell. Thus, the first lattice 110 shown in Figure 6 may be characterized as closed-cell. In contrast, the second lattice 112 may be characterized as open-cell.

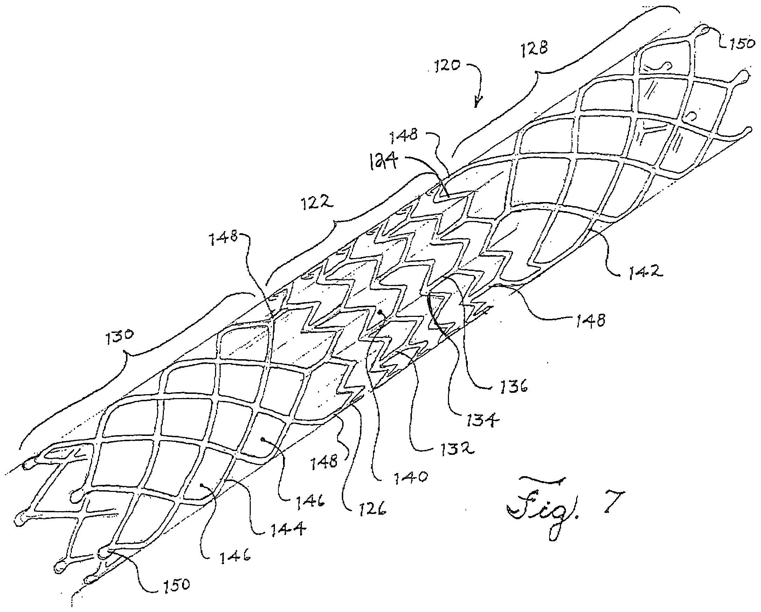

In another illustrative constructions, the multi-section tubular device 120 may combine a central open-cell lattice with two closed-cell lattices on either side as shown in Figure 7. In such an construction, the tubular device 120 may include a first tubular section 122 having a first end 124 and a second end 126. A second tubular section 128 may be connected to the first end 124 of the first tubular section 122. A third tubular section 130 may be connected to the second end 126 of the first tubular section 122. In this construction, the first tubular section 122 may form a first lattice 132 having ring segments 134 that extend circumferentially in a zigzag pattern and are joined together by at least three substantially longitudinally running connector segments 136. As shown in Figure 7, the longitudinal length of the cells 140 is less than the circumferential length of the cells 140 in the lattice 132 of the first tubular section 122. Thus, the first lattice 132 may be characterized as being open-cell. The second tubular section 128 and the third tubular section 130 form a second 142 and third lattice 144, respectively. In the second lattice 142 and the third lattice 144, the longitudinal length of the cells 146 is substantially equal to the circumferential length. Accordingly, the second 142 and third lattices 144 may be characterized as being closed-cell lattices.

The first tubular section 122 is connected to the surrounding second and third tubular section in Figure 7 by three longitudinally running connector segments 148. Although the connector segments shown in Figure 7 run longitudinally, other embodiments are possible in which the connector segments run substantially longitudinally.

The tubular device shown in Figure 7 may feature the use of a radio-opaque substance both in the rounded tips 150 extending from the ends of the second 128 and third tubular sections 130 and along the junctions between the first 122 and second tubular sections 128 and the first 122 and third tubular sections 130. The rounded tips 150 preferably extend from the ends of the second 128 and third tubular sections 130 that are not attached to the first tubular section 122. A radio-opaque substance, such as gold may be embedded in the rounded tips 150. In constructions using a radio-opaque substance between sections, the substance is preferably located within one of the connector segments 148 linking two adjacent sections of the multi-section tubular device 120. Some constructions may also feature a radio-opaque coating in lieu of, or in conjunction with, embedded radio-opaque material.

The combination of open-cell and closed-cell lattices shown in Figure 7 may be advantageous because the two closed-cell lattices of the second 128 and third tubular sections 130 will provide good plaque coverage and high radial strength. The open-cell lattice of the first tubular section 122 will provide the tubular device with flexibility so that the stent may withstand axial, radial, and torsional forces without kinking or fracturing.

The multi-section tubular device is preferably made of nitinol (nickel and titanium). However, the device may also be made of stainless steel, cobalt chromium, or any biocompatible material known in the art. The tubular device may be made of a single material or a combination of any of the aforementioned materials.

The length of the individual sections of the multi-section tubular device may vary depending on the blood vessel in which the device is used. For example, blood vessels in the knee may require a long flexible first section. In contrast, the blood vessels in the ankle that are subject to high quantities and different varieties of force are relatively short. Thus, the first tubular section of a multi-section tubular device for use in a blood vessel of the ankle may be shorter than the first tubular section of a multi-section tubular device intended to be used in the knee.

All or part of the multi-section tubular device may also be made of a bioabsorbable material. In particular, it may be preferable for embodiments similar to the embodiment shown in Figure 5 have additional connector segments in the first tubular section 62 made of a bioabsorbable material. Once the stent is inserted, these additional bioabsorbable connector segments may be absorbed by the body, thereby reducing the overall number of connector segments in the first tubular section 62, leaving as few as three segments. This may be beneficial because a high number of connector segments may aid in maximizing radial force and strength during insertion into the blood vessel. Once the tubular device is in place, the reduction in the overall number of connector segments may maximize the flexibility of the first tubular section 62.

Multi-section tubular devices may also feature the addition of a graft material covering any one of the tubular sections. For example, in the example of a tubular device depicted in Figure 3, it may be advantageous to add a graft material over the coiled first tubular section 40. Preferably, the graft material is slightly longer than the coiled first tubular section 40 when the coiled first tubular is in a non-compressed state. For example, it may be preferable to have graft material that runs from the third row of cells 44 of the second tubular section to the third row of cells 44 of the third tubular section. However, the graft preferably does not cover the entire multi-section tubular body. In some embodiments, the graft material may cover the first tubular section only, or a combination of the first tubular section and a portion of the other sections. In other embodiments it may cover only one of the second and third tubular sections. In still other embodiments the graft material may cover only part of the first tubular section. Any other suitable configuration of the graft material may be used, depending on blood vessel in which the stent-graft is being inserted.

In embodiments featuring the addition of graft material, the graft material is preferably an impermeable material. Suitable materials include woven polyesters such as Dacron. Any other graft material known in the art may also be used. The graft material may be located over any desirable section of the multi-section tubular body.

The graft material is preferably not attached to the entire length of the multi-section tubular body covered by the graft. Instead, the graft material is preferably attached to the second tubular section and the third tubular section only. This may be advantageous because the graft material will not restrict the movement of the flexible first tubular section. This is particularly advantageous in illustrative constructions having a coiled first tubular section because the coiled tubular section may increase in length as it is twisted to be loaded into the stent delivery device. The loose graft material will not restrict the first tubular section as it increases in length for loading and decreases in length when the stent is deployed. The graft material may be attached to the stent through sutures or any other technique known in the art.

It may also be advantageous to have graft material that does not cover the entire length of the multi-section tubular body because it allows at least a portion of the multi-section tubular body to directly contact the interior of the blood vessel. Over time, the tissue of the blood vessel may grow into the exposed cells of the stent to secure the stent firmly in place. For example, in some embodiments having a graft material, the ends of the second and third tubular bodies may not be covered by the graft material. These ends may then serve to anchor the stent-graft in the blood vessel as the blood vessel grows around the stent.

Tubular devices featuring a graft material are particularly suitable for treating aneurysms. The graft material may prevent blood from flowing into the damaged portion of the vessel. The use of a multi-section tubular device with graft material is particularly advantageous for use in treating aneurysms in blood vessels subject to a wide range of different axial, torsional, and radial forces.

For embodiments of the multi-section tubular device that are composed of metals, the lattice is preferably laser cut from a single metallic tube. In this preferred embodiment, the stent is formed of one integral unit. In other embodiments, the three tubular sections of the tubular device may be laser cut separately and then connected together by welding, sutures, a combination of grafts and sutures, or any other technique known in the art. In still other embodiments, the lattice may be formed of wire. The wire may be twisted into the desired tubular shape and then heat set so that the wire retains that shape. Any heat-setting technique known in the art may be used for such embodiments. The tubular device may be either self-expanding or balloon expandable.

It is preferable that the shape of the individual metallic segments or wire have a flat shape. The edges of the segments or wire are preferably smoothed to eliminate any sharp edges that might damage the walls of a blood vessel. The flat shape maximizes the area of the blood vessel on which the tubular device exerts radial force. Although a flat shape is preferable, a round shape may also be used.

The multi-section tubular device is for use in the superficial femoral artery (SFA).

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A multi-section stent for use in the superficial femoral artery, the stent comprising: a first tubular section (62) having a first end (64) and a second end (66); a second tubular section (68) connected to said first end (64) of said first tubular section (62); and a third tubular section (70) connected to said second end (66) of said first tubular section (62), wherein each tubular section (62, 68, 70) comprises at least one segment defining at least one cell that extends longitudinally and circumferentially of the stent, wherein the ratio of the circumferential length to the longitudinal length of said at least one cell is greater in said first tubular section (62) than in either of said second tubular section (68) or said third tubular section (70), and wherein each of said first tubular section (62), said second tubular section (68), and said third tubular section (70) is formed of a lattice (72, 74, 76), said lattices each having a plurality of ring segments (80) extending circumferentially and at least three connector segments (84) linking each of said ring segments (80) together with each adjacent ring segment, wherein said first tubular section (62) has a substantially lower ratio of said connector segments (84) to said ring segments (80) than said second tubular section (68), and said first tubular section (62) has a substantially lower ratio of said connector segments (84) to said ring segments (80) than said third tubular section (70).

2. The multi-section tubular stent of Claim 1, wherein the longitudinal length of said first tubular section is greater than the longitudinal length of said second tubular section and the longitudinal length of said third tubular section.

3. The multi-section tubular stent of Claim 1 or Claim 2, further comprising a radio-opaque substance contained on at least one of said at least substantially longitudinally running connector segments linking two adjacent sections of said multi-section tubular device together.

4. The multi-section tubular stent of Claim 1 or Claim 2, wherein three connector segments connect each of said ring segments in said first tubular section to each adjacent ring segment in said first tubular section.

5. The multi-section stent of any one of claims 1 to 4, comprising additional connector segments made of a bioabsorbable material.

6. The multi-section stent according to claim 1, wherein said first lattice comprises a plurality of cells, each cell having a circumferential length and a longitudinal length, said circumferential length of each cell of said first lattice being substantially greater than said longitudinal length and said second lattice and third lattice each comprise a plurality of cells, each cell having a circumferential length and a longitudinal length, said circumferential length of each cell of said second and third lattice being substantially equal to said longitudinal length.

7. The multi-section stent according to Claim 6, wherein said first lattice of said first tubular section further comprises: a plurality of ring segments extending circumferentially in a zigzag pattern; and a plurality of at least substantially longitudinally-running connector segments linking adjacent ring segments together in a longitudinal direction, wherein said first end of said first tubular section is connected to said second tubular section by at least one of said at least substantially longitudinally-running connector segments and said second end of said first tubular section is connected to said third tubular section by at least one of said at least substantially longitudinally-running connector segments.

8. The multi-section stent according to Claim 1, wherein the number of connector segments in said first tubular section decreases towards the centre of said first tubular section.

9. The multi-section stent according to Claim 1, wherein the connector segments of said first tubular section are disposed in a spiral pattern.

10. The multi-section stent according to any one of the preceding claims,
wherein said first tubular section, said second tubular section, and said third tubular section are nitinol.

11. The multi-section stent according to any one of the preceding claims,
wherein said multi-section tubular device is integral.

12. The multi-section stent according to any one of the preceding claims, further comprising a graft material covering at least a portion of at least one section of said multi-section tubular device.

## Patentansprüche

1. Stent mit mehreren Zonen zur Verwendung in der oberflächlichen Oberschenkelarterie, wobei der Stent Folgendes umfasst: eine erste röhrenförmige Zone (62) mit einem ersten Ende (64) und einem zweiten Ende (66); eine zweite röhrenförmige Zone (68), die mit dem ersten Ende (64) der ersten röhrenförmigen Zone (62) verbunden ist; und eine dritte röhrenförmige Zone (70), die mit dem zweiten Ende (66) der ersten röhrenförmigen Zone (62) verbunden ist, wobei jede röhrenförmige Zone (62, 68, 70) mindestens ein Segment umfasst, das mindestens eine Zelle definiert, die sich längs und um den Umfang des Stents erstreckt, wobei das Verhältnis der Umfangslänge zur Längslänge der mindestens einen Zelle in der ersten röhrenförmigen Zone (62) größer als in der zweiten röhrenförmigen Zone (68) oder in der dritten röhrenförmigen Zone (70) ist, und wobei die erste röhrenförmige Zone (62), die zweite röhrenförmige Zone (68) und die dritte röhrenförmige Zone (70) jeweils aus einem Gitter (72, 74, 76) gebildet sind, wobei die Gitter jeweils eine Vielzahl von Ringsegmenten (80), die sich um den Umfang erstrecken, und mindestens drei Verbindungssegmente (84) aufweisen, die jedes der Ringsegmente (80) zusammen mit jedem benachbarten Ringsegment verbinden, wobei die erste röhrenförmige Zone (62) ein wesentlich niedrigeres Verhältnis der Verbindungssegmente (84) zu den Ringsegmenten (80) aufweist als die zweite röhrenförmige Zone (68), und wobei die erste röhrenförmige Zone (62) ein wesentlich niedrigeres Verhältnis der Verbindungssegmente (84) zu den Ringsegmenten (80) als die dritte röhrenförmige Zone (70) aufweist.

2. Röhrenförmiger Stent mit mehreren Zonen nach Anspruch 1, wobei die Längslänge der ersten röhrenförmigen Zone größer als die Längslänge der zweiten röhrenförmigen Zone und die Längslänge der dritten röhrenförmigen Zone ist.

3. Röhrenförmiger Stent mit mehreren Zonen nach Anspruch 1 oder Anspruch 2, ferner umfassend eine röntgenopake Substanz, die auf mindestens einem der mindestens im Wesentlichen längs verlaufenden Verbindungssegmente, die zwei benachbarte Zonen der röhrenförmigen Vorrichtung mit mehreren Zonen zusammen verbinden, enthalten ist.

4. Röhrenförmiger Stent mit mehreren Zonen nach Anspruch 1 oder Anspruch 2, wobei drei Verbindungssegmente jedes der Ringsegmente in der ersten röhrenförmigen Zone mit jedem benachbarten Ringsegment in der ersten röhrenförmigen Zone verbinden.

5. Stent mit mehreren Zonen nach einem der Ansprüche 1 bis 4, umfassend zusätzliche Verbindungssegmente, die aus einem bioresorbierbaren Material gefertigt sind.

6. Stent mit mehreren Zonen nach Anspruch 1, wobei das erste Gitter eine Vielzahl von Zellen umfasst, wobei jede Zelle eine Umfangslänge und eine Längslänge aufweist, wobei die Umfangslänge jeder Zelle des ersten Gitters wesentlich größer als die Längslänge ist und das zweite Gitter und das dritte Gitter jeweils eine Vielzahl von Zellen umfassen, wobei jede Zelle eine Umfangslänge und eine Längslänge aufweist, wobei die Umfangslänge jeder Zelle des zweiten und dritten Gitters im Wesentlichen der Längslänge gleicht.

7. Stent mit mehreren Zonen nach Anspruch 6, wobei das erste Gitter der ersten röhrenförmigen Zone ferner Folgendes umfasst: eine Vielzahl von Ringsegmenten, die sich um den Umfang in einem Zickzack-Muster erstrecken; und eine Vielzahl von mindestens im Wesentlichen längs verlaufenden Verbindungssegmenten, die benachbarte Ringsegmente zusammen in einer Längsrichtung verbinden, wobei das erste Ende der ersten röhrenförmigen Zone mit der zweiten röhrenförmigen Zone durch mindestens eines der mindestens im Wesentlichen längs verlaufenden Verbindungssegmente verbunden ist und das zweite Ende der ersten röhrenförmigen Zone mit der dritten röhrenförmigen Zone durch mindestens eines der mindestens im Wesentlichen längs verlaufenden Verbindungssegmente verbunden ist.

8. Stent mit mehreren Zonen nach Anspruch 1, wobei die Anzahl der Verbindungssegmente in der ersten röhrenförmigen Zone zur Mitte der ersten röhrenförmigen Zone hin abnimmt.

9. Stent mit mehreren Zonen nach Anspruch 1, wobei die Verbindungssegmente der ersten röhrenförmigen Zone in einem Spiralmuster angeordnet sind.

10. Stent mit mehreren Zonen nach einem der vorhergehenden Ansprüche, wobei die erste röhrenförmige Zone, die zweite röhrenförmige Zone und die dritte röhrenförmige Zone aus Nitinol bestehen.

11. Stent mit mehreren Zonen nach einem der vorhergehenden Ansprüche, wobei die röhrenförmige Vorrichtung mit mehreren Zonen einstückig ist.

12. Stent mit mehreren Zonen nach einem der vorhergehenden Ansprüche, ferner umfassend ein Transplantatmaterial, das mindestens einen Teil mindestens einer Zone der röhrenförmigen Vorrichtung mit mehreren Zonen bedeckt.

## Revendications

1. Endoprothèse vasculaire à plusieurs sections à utiliser dans l'artère fémorale superficielle, l'endoprothèse comprenant :
une première section (62) tubulaire comportant une première extrémité (64) et une seconde extrémité (66) ;
une deuxième section (68) tubulaire raccordée à ladite première extrémité (64) de ladite première section (62) tubulaire ; et
une troisième section (70) tubulaire raccordée à ladite seconde extrémité (66) de ladite première section (62) tubulaire,
dans laquelle chaque section (62, 68, 70) tubulaire comporte au moins un segment définissant au moins une cellule qui s'étend suivant la longueur et la circonférence de l'endoprothèse vasculaire,
dans laquelle le rapport de la dimension de la circonférence sur la dimension de la longueur de ladite cellule est plus élevé dans ladite première section (62) tubulaire que dans l'une ou l'autre desdites deuxième section (68) tubulaire et troisième section (70) tubulaire, et
dans laquelle chacune desdites première section (62) tubulaire, deuxième section (68) tubulaire et troisième section (70) tubulaire est constituée d'un treillis (72, 74, 76), lesdits treillis comportant chacun une pluralité de segments annulaires (80) s'étendant suivant la circonférence et au moins trois segments de raccord (84) reliant chacun desdits segments annulaires (80) à chaque segment annulaire adjacent,
dans laquelle ladite première section (62) tubulaire présente un ratio desdits segments de raccord (84) par rapport auxdits segments annulaires (80) sensiblement inférieur à celui de ladite deuxième section (68) tubulaire, et ladite première section (62) tubulaire présente un ratio desdits segments de raccord (84) par rapport auxdits segments annulaires (80) sensiblement inférieur à celui de ladite troisième section (70) tubulaire.

2. Endoprothèse vasculaire tubulaire à plusieurs sections selon la revendication 1, dans laquelle la dimension longitudinale de ladite première section tubulaire est supérieure à la dimension longitudinale de ladite deuxième section tubulaire et à la dimension longitudinale de ladite troisième section tubulaire.

3. Endoprothèse vasculaire tubulaire à plusieurs sections selon la revendication 1 ou 2, comprenant en outre une substance radio-opaque contenue sur au moins un desdits segments de raccord au parcours sensiblement longitudinal reliant l'une à l'autre deux sections adjacentes dudit dispositif tubulaire à plusieurs sections.

4. Endoprothèse vasculaire tubulaire à plusieurs sections selon la revendication 1 ou 2, dans laquelle trois segments de raccord raccordent chacun desdits segments annulaires dans ladite première section tubulaire à chaque segment annulaire adjacent dans ladite première section tubulaire.

5. Endoprothèse vasculaire à plusieurs sections selon l'une quelconque des revendications 1 à 4, comprenant des segments de raccord supplémentaires faits d'une matière bioabsorbable.

6. Endoprothèse vasculaire à plusieurs sections selon la revendication 1, dans laquelle ledit premier treillis comprend une pluralité de cellules, chaque cellule ayant une dimension circonférentielle et une dimension longitudinale, ladite dimension circonférentielle de chaque cellule dudit premier treillis étant sensiblement supérieure à ladite dimension longitudinale et lesdits deuxième treillis et troisième treillis comprennent chacun une pluralité de cellules, chaque cellule ayant une dimension circonférentielle et une dimension longitudinale, ladite dimension circonférentielle de chaque cellule desdits deuxième et troisième treillis étant sensiblement égale à ladite dimension longitudinale.

7. Endoprothèse vasculaire à plusieurs sections selon la revendication 6, dans laquelle ledit premier treillis de ladite première section tubulaire comprend en outre :
une pluralité de segments annulaires s'étendant suivant la circonférence dans une configuration à zigzags ; et
une pluralité de segments de raccord au parcours au moins sensiblement longitudinal reliant l'un à l'autre des segments annulaires adjacents dans la direction longitudinale,
dans laquelle ladite première extrémité de ladite première section tubulaire est raccordée à ladite deuxième section tubulaire par au moins un desdits segments de raccord au parcours au moins sensiblement longitudinal et ladite seconde extrémité de ladite première section tubulaire est raccordée à ladite troisième section tubulaire par au moins un desdits segments de raccord au parcours au moins sensiblement longitudinal.

8. Endoprothèse vasculaire à plusieurs sections selon la revendication 1, dans laquelle le nombre de segments de raccord dans ladite première section tubulaire diminue vers le centre de ladite première section tubulaire.

9. Endoprothèse vasculaire à plusieurs sections selon la revendication 1, dans laquelle les segments de raccord de ladite première section tubulaire sont disposés en spirale.

10. Endoprothèse vasculaire à plusieurs sections selon l'une quelconque des revendications précédentes, dans laquelle ladite première section tubulaire, ladite deuxième section tubulaire et ladite troisième section tubulaire sont en nitinol.

11. Endoprothèse vasculaire à plusieurs sections selon l'une quelconque des revendications précédentes, dans laquelle ledit dispositif tubulaire à plusieurs sections est d'un seul tenant.

12. Endoprothèse vasculaire à plusieurs sections selon l'une quelconque des revendications précédentes, comprenant en outre une matière de greffon couvrant au moins une partie d'au moins une section dudit dispositif tubulaire à plusieurs sections.
